# EUROPEAN PATENT SPECIFICATION

(11) **EP 2 454 386 B1**
(45) Date of publication and mention of the grant of the patent: **16.09.2015**
(21) Application number: 10800124.9
(22) Date of filing: 13.07.2010
(51) Int. Cl.: C12Q 1/68, C12N 15/44, C12N 15/11, C12Q 1/70

(54) **INFLUENZA DETECTION METHOD AND KIT THEREFOR**
INFLUENZA-ERKENNUNGSVERFAHREN UND KIT DAFÜR
PROCÉDÉ DE DÉTECTION DE GRIPPE ET TROUSSE POUR CE FAIRE

(30) Priority: 13.07.2009 SG 200904777
(43) Date of publication of application: 23.05.2012
(73) Proprietor: Agency for Science, Technology and Research, Singapore 138632 (SG); Tan Tock Seng Hospital Pte Ltd, Singapore 308433 (SG)
(72) Inventor: INOUE, Masafumi, Singapore 138669 (SG); BARKHAM, Timothy, Singapore 308433 (SG)
(74) Representative: Fleck, Barbara
(86) International application number: PCT/SG2010/000263
(87) International publication number: WO 2011/008171

(56) References cited:
- WO-A1-94/21797
- WO-A1-2007/099451
- WO-A2-2007/017759
- WO-A2-2007/018563
- WO-A2-2007/053696
- WO-A2-2007/058629
- WO-A2-2007/058629
- WO-A2-2008/054830
- US-A- 6 015 664
- US-A- 6 015 664
- US-A1- 2005 221 354
- US-B2- 7 250 289
- US-B2- 7 312 035
- US-B2- 7 314 750
- US-B2- 7 374 927
- BOLOTIN S ET AL: "Development of a novel real-time reverse-transcriptase PCR method for the detection of H275Y positive influenza A H1N1 isolates", JOURNAL OF VIROLOGICAL METHODS, ELSEVIER BV, NL, vol. 158, no. 1-2, 1 January 2009 (2009-01-01), pages 190-194, XP008156800, ISSN: 0166-0934
- KESSLER N ET AL: "Use of the DNA flow-thru chip, a three dimensional biochip, for typing and subtyping influenza viruses", JOURNAL OF CLINICAL MICROBIOLOGY, AMERICAN SOCIETY FOR MICROBIOLOGY, WASHINGTON, DC, US, vol. 42, no. 5, 1 January 2004 (2004-01-01), pages 2173-2185, XP003004496, ISSN: 0095-1137, DOI: 10.1128/JCM.42.5.2173-2185.2004
- MEIJER A ET AL: "Preparing the outbreak assistance laboratory network in the Netherlands for the detection of the influenza virus A(H1N1) variant", JOURNAL OF CLINICAL VIROLOGY, ELSEVIER, AMSTERDAM, NL, vol. 45, no. 3, 1 July 2009 (2009-07-01), pages 179-184, XP026236981, ISSN: 1386-6532, DOI: 10.1016/J.JCV.2009.06.003 [retrieved on 2009-06-11]
- SUWANNAKARN K ET AL: "Typing (A/B) and subtyping (H1/H3/H5) of influenza A viruses by multiplex real-time RT-PCR assays", JOURNAL OF VIROLOGICAL METHODS, ELSEVIER BV, NL, vol. 152, no. 1-2, 1 September 2008 (2008-09-01), pages 25-31, XP023315642, ISSN: 0166-0934, DOI: 10.1016/J.JVIROMET.2008.06.002 [retrieved on 2008-07-15]
- DI TRANI LIVIA ET AL: "A sensitive one-step real-time PCR for detection of avian influenza viruses using a MGB probe and an internal positive control", BMC INFECTIOUS DISEASES, BIOMED CENTRAL, LONDON, GB, vol. 6, no. 1, 25 May 2006 (2006-05-25), page 87, XP021015749, ISSN: 1471-2334, DOI: 10.1186/1471-2334-6-87
- WHILEY ET AL: "A 5'-nuclease real-time reverse transcriptase-polymerase chain reaction assay for the detection of a broad range of influenza A subtypes, including H5N1", DIAGNOSTIC MICROBIOLOGY AND INFECTIOUS DISEASES, ELSEVIER SCIENCE PUBLISHING CO., AMSTERDAM, NL, vol. 53, no. 4, 1 December 2005 (2005-12-01), pages 335-337, XP005212901, ISSN: 0732-8893, DOI: 10.1016/J.DIAGMICROBIO.2005.08.002

## Description

### FIELD OF THE INVENTION

The present invention relates to primer(s), probes as well as method(s) and kit(s) using such primer(s) and/or probes for the detection of the presence of Influenza A and/or B virus.

### BACKGROUND TO THE INVENTION

There are in total three types of Influenza virus strains- A, B and C. Out of the three strains, Influenza A is considered to be the most virulent as it can infect a variety of animals including human beings. Influenza B and influenza C viruses normally only infect human beings. Influenza viruses that use wild birds as natural hosts are referred to as "avian influenza viruses" and influenza viruses that use human beings as natural hosts are referred to as "human influenza viruses". In several cases, human beings and other animals become infected with avian influenza viruses through contact with infected domesticated birds and/or infected wild birds. Avian influenza viruses that have crossed species and infected human beings have been responsible for the recent human influenza pandemics.

The influenza virus, an enveloped virus has a genome containing eight single-stranded negative sense RNA segments. The viral envelope has a host-derived lipid bilayer with two major surface viral glycoproteins: hemagglutinin ("HA") and neuraminidase ("NA"), which are the proteins responsible for viral attachment. Within the envelope, matrix protein ("MI") and nucleoprotein ("NP") protect the viral RNA. The A, B, and C type designation of the influenza virus is based upon the antigenic features of the MI matrix protein and NP. The eight RNA segments encode at least 10 viral proteins: segments 1, 2, and 3 encode three viral polymerase proteins; segment 4 encodes HA; segment 5 encodes NP; segment 6 encodes NA; segment 7 encodes the M1 and M2 matrix proteins, the former which has ion channel activity and is embedded in the viral envelope; and segment 8 encodes the nonstructural proteins NS1 and NS2, the former which blocks the hosts antiviral response and the latter which participates in the assembly of virus particles.

The influenza A strain can be further divided into subtypes based on the HA and NA proteins on the surface of the virus. The influenza A virus has 16 different HA subtypes and 9 different NA subtypes, all of which may exist on the surface of the virus in many different combinations. For example, a H5N1 virus has an HA5 protein and an NA1 protein on its surface. The reason for the high degree of genetic and hence immunological variability especially of the influenza A viruses is due to the fact that a genetic shift (reassortment of the viral genes) can also occur in rare cases in addition to the usual genetic drift (point mutation). This is due to the fact that, in contrast to other viruses, the genome of the influenza viruses is segmented and that influenza A is a pathogen in human beings as well as in animals.

All the subtypes have been found in birds. The more common influenza A subtypes found in human beings are the H1, H2, and H3 subtypes with the H5, H7, and H9 subtypes also having been known to infect human beings. The influenza B and C strains are not classified according to subtype.

Since 1997, influenza A viruses previously exclusive to infection in birds have been infecting humans with fatal outcomes. Confirmed outbreaks of avian influenza virus with some resultant human deaths have been reported in 1997 (H5N1 in Hong Kong), 1999 (H9N2 in China and Hong Kong), 2002 (H7N2 in Virginia, USA), 2003 (H5N1 in China and Hong Kong; H7N7 in the Netherlands; H9N2 in Hong Kong; H7N2 in New York, USA); 2004 (H5N1 in Thailand and Vietnam; H7N3 in Canada); and 2005 (H5N1 in Thailand and Vietnam).

The influenza A and B viruses primarily infect the nasopharyngeal and oropharyngeal cavities and initially cause general respiratory symptoms in the affected persons. It is not possible even for experienced medical professionals to very reliably diagnose influenza solely on the basis of the patient's clinical symptoms since other viruses which infect the nasal or pharyngeal cavity such as adenoviruses, parainfluenza viruses or respiratory syncitial viruses (RS viruses) cause similar symptoms. Traditional methods to detect avian influenza A and B viruses include plaque assays, such as Culture Enhanced Enzyme Linked Immunosorbent Assay ("CE-ELISA") and virus isolation in embryonated chicken eggs. Hemagglutinin and neuraminidase subtyping of the virus is carried out after detection by serological methods. While the traditional methods have been shown to be sufficiently sensitive, the processes are time-consuming and is thus not of immediate relevance for the diagnosis of individual patients; for example, virus isolation in embryonated eggs takes from one to two weeks to obtain results. Other methods of detecting influenza A/B infections involve antigen detection, isolation in cell culture, or detection of influenza-specific RNA by reverse transcriptase polymerase chain reaction (RT-PCR). There is a huge problem in using antigen detection due to the sensitivity of such tests. The rapid test kits generally provide results within 24 hours and are approximately 70% sensitive for detecting influenza and approximately 90% specific. The sensitivity of the rapid test kits means that as many as 30% of samples may yield false negatives, and the tests are not multiplexed. They are all conducted in a laboratory and usually results are not produced rapidly enough to impact on the prescribed treatment.

WO 2007/058629 discloses the use of primers and probes for the detection of influenza A subtype H5N1 by PCR using the Luminex platform for DNA hybridization.

US 6015664 discloses multiplex PCR assay using oligonucleotide primers for the detection of multiple virus infections, which include HPIV, RSV and influenza A and B.

WO 2007/053696 discloses compositions and methods for modulating expression of influenza viral genes and the down regulation of influenza viral genes by chemically modified oligonucleotides.

WO 2007/017759 discloses genetically modified animals which are resistant to viral infection, including influenza and sub-type avian influenza H5N1, and oligonucleotide sequences that form part of the H5N1 genome.

US 7374927 B2 provides arrays for analysis of compromised nucleic acid samples and discloses a large number of oligonucleotides.

WO 2007/099451 describes a method for identifying useful proteins of brewery yeast and discloses a large number of oligonucleotides.

US 7312035 describes a method for identifying useful proteins in brewery yeast and genes encoding the proteins thereof and discloses a large number of oligonucleotides.

US 7250289 describes an array of oligonucleotide probes which may be used to measure the expression of a plurality of genes simultaneously and discloses a large number of oligonucleotides.

US 2005/0221354 discloses an array of nucleic acid sequences which are complementary to a wide variety of mouse genes and discloses a large number of oligonucleotides.

US 7314750 discloses an array of nucleic acids and methods of using these arrays for detecting or monitoring expression profiles of drug target genes.

WO 2008/054830 describes an array of nucleic acids complementary to rat genes and a methods of expression of said genes and discloses a large number of oligonucleotides.

WO 1994/021797 discloses DNA constructs for expression of influenza gene products in animals to raise an immune response.

WO 2007/018563 teaches probe arrays for detecting multiple strains of viral and non-viral species, including influenzas A, B and C.

Bolotin et al; Journal of Virological Methods; vol. 158; 2009, discloses a real-time reverse-transcriptase PCR assay for the detection of the H275Y mutation in influenza A H1N1 isolates, including oligonucleotide primers and probes for use in the assay. Kessler et al; Journal of Clinical Microbiology; vol. 42; 2004, discloses a method for the typing and subtyping of influenza viruses using a three-dimensional biochip platform, including oligonucleotide probes and primers for use in the method.

Meijer et al; Journal of Clinical Virology; vol. 45; 2009, describes the preparation of laboratories for surge capacity for molecular diagnosis of patients suspected for Avian H1N1 virus infection in the Netherlands, including specific primers and probes for use in a test protocol.

Suwannakarn et al; Journal of Virological Methods; vol. 152; 2008, discloses typing (A/B) and subtyping (H1/H3/H5) of influenza A viruses by multiplex real-time RT-PCR assays.

Trani et al; BMC Infectious Diseases; vol. 6; 2006 describes the design of a one-step reverse transcription real time PCR for the detection of different subtypes of AIVs, and the primers and probes used in this method.

Whiley et al; Diagnostic Microbiology and Infectious Disease; vol. 53; 2005, describes a PCR based assay for the detection of influenza A subtypes.

Currently as stipulated by the World Health Organization, recommended diagnostic methods for the identification of avian influenza A virus in human are immunofluorescence assay (IFA), virus culture and PCR assays. Both IFA and virus culture methods are laborious and are not feasible for large amounts of samples.

### SUMMARY OF THE INVENTION

The present invention is defined in the appended independent claims. Some optional features of the present invention are defined in the appended dependent claims.

In particular, the present disclosure addresses the problems above, and provides highly sensitive and specific oligonucleotides, fragments and/or derivatives thereof useful in a method of detecting influenza virus in patient specimens more efficiently.

The primers and/or probes may be sensitive and specific in the detection of influenza and provide rapid and cost-effective diagnostic and prognostic reagents for determining infection by influenza and/or disease conditions associated therewith. In particular, these primers provide a specific and/or sensitive detection method capable of detecting all influenza A and influenza B strains and subtypes by differentiating seasonal influenza A and B from novel influenza A as well as detecting newly emergent influenza A and B strains. These primers may provide an informative influenza assay that can be performed in the field, i.e., at the point of care ("POC").

According to a first aspect the present invention provides a kit for multiplex detection of influenza A or influenza A and influenza B virus in a biological sample, the kit comprising a mixture of primers and probes said mixture comprising a set of oligonucleotides comprising at least one forward primer and at least one reverse primer and at least one probe, wherein the forward primer comprises the nucleotide sequence of SEQ ID NO:1, the reverse primer comprises the nucleotide sequence of SEQ ID NO:2 and the probe comprises the nucleotide sequence of SEQ ID NO:3, and said mixture further comprising one or more sets of oligonucleotides selected from the group comprising:
a. a forward primer comprising the nucleotide sequence of SEQ ID NO: 4 and a reverse primer comprising the nucleotide sequence of SEQ ID NO: 5 and at least one probe comprising SEQ ID NO:6 to detect swine-origin H1N1;
b. a forward primer comprising the nucleotide sequence of SEQ ID NO: 7 and a reverse primer comprising the nucleotide sequence of SEQ ID NO: 8 and at least one probe comprising SEQ ID NO:9 to detect swine-origin H1N1;
c. a forward primer comprising the nucleotide sequence of SEQ ID NO: 10 and a reverse primer comprising the nucleotide sequence of SEQ ID NO: 11 and at least one probe comprising SEQ ID NO:12 to detect seasonal H1N1;
d. a forward primer comprising the nucleotide sequence of SEQ ID NO: 13 and a reverse primer comprising the nucleotide sequence of SEQ ID NO: 14 and at least one probe comprising SEQ ID NO:15 to detect seasonal H3N2, and
e. a forward primer comprising the nucleotide sequence of SEQ ID NO: 16 and a reverse primer comprising the nucleotide sequence selected from SEQ ID NO: 17 and 18 and at least one probe comprising or consisting of SEQ ID NO:19 to detect influenza B.

According to one aspect, the present disclosure provides at least one isolated oligonucleotide comprising, consisting essentially of, or consisting of at least one nucleotide sequence selected from the group consisting of: SEQ ID NO:1 to SEQ ID NO:19, fragment(s), derivative(s), mutation(s), and complementary sequence(s) thereof. The oligonucleotide may be capable of binding to and/or being amplified from influenza A and/or B virus.

According to another aspect, the present disclosure provides at least one pair of oligonucleotides comprising at least one forward primer and at least one reverse primer, wherein the forward primer comprises, consists essentially of or consists of at least one nucleotide sequence selected from the group consisting of SEQ ID NO:1, SEQ ID NO:4, SEQ ID NO:7, SEQ ID NO:10, SEQ ID NO:13, SEQ ID NO:16, fragment(s), derivative(s), mutation(s), and complementary sequence(s) thereof and the reverse primer comprises, consists essentially of or consists of at least one nucleotide sequence selected from the group consisting of SEQ ID NO:2, SEQ ID NO:5, SEQ ID NO:8, SEQ ID NO:11, SEQ ID NO:14, SEQ ID NO:17, SEQ ID NO:18, fragment(s), derivative(s), mutation(s), and complementary sequence(s) thereof.

According to another aspect, the present disclosure provides at least one set of oligonucleotides comprising a pair of oligonucleotides according to any aspect of the present disclosure and at least one probe. The probe comprises, consists essentially of or consists of a nucleotide sequence that may be selected from the group consisting of SEQ ID NO:3, SEQ ID NO:6, SEQ ID NO:9, SEQ ID NO:12, SEQ ID NO:15, SEQ ID NO:19, fragment(s), derivative(s), mutation(s), and complementary sequence(s) thereof.

According to a further aspect, the present disclosure provides at least one amplicon amplified from influenza A and/or B virus using at least one forward primer comprising, consisting essentially of or consisting of the nucleotide sequence selected from the group consisting of SEQ ID NO:1, SEQ ID NO:4, SEQ ID NO:7, SEQ ID NO:10, SEQ ID NO:13 and SEQ ID NO:16, fragment(s), derivative(s), mutation(s), and complementary sequence(s) thereof and at least one reverse primer comprising, consisting essentially of or consisting of the nucleotide sequence from the group consisting of SEQ ID NO:2, SEQ ID NO:5, SEQ ID NO:8, SEQ ID NO:11, SEQ ID NO:14, SEQ ID NO:17, SEQ ID NO:18, fragment(s), derivative(s), mutation(s), and complementary sequence(s) thereof.

In another aspect, the invention relates to an in vitro method of detecting and/or quantitating the presence of influenza A in a biological sample, the method comprising the steps of:
(a) providing at least one biological sample;
(b) contacting a mixture of primers and probes as defined in the first aspect of the invention with at least one nucleic acid in the biological sample, and/or with at least one nucleic acid extracted, purified and/or amplified from the biological sample; and
(c) detecting and/or quantitating any binding resulting from the contacting in step (b) whereby the virus is present when binding is detected.

In another aspect, the invention relates to an in vitro method of detecting and/or quantitating the presence of Influenza A and B virus in a biological sample, the method comprising the steps of:
(a) providing at least one biological sample;
(b) contacting a mixture of primers and probes according to the first aspect of the invention, with at least one nucleic acid in the biological sample, and/or with at least one nucleic acid extracted, purified and/or amplified from the biological sample; and
(c) detecting and/or quantitating any binding resulting from the contacting in step (b) whereby the virus is present when binding is detected.

According to one aspect, the present disclosure provides at least one method of amplifying influenza A and/or B virus nucleic acid, wherein said method comprises carrying out a polymerase chain reaction using at least one forward primer according to any aspect of the present invention and a reverse primer according to any aspect of the present invention.

According to another aspect, the present disclosure provides at least one kit for the detection of influenza A and/or B virus, the kit comprising at least one oligonucleotide, pair of oligonucleotides or set of oligonucleotides according to any aspect of the present disclosure. All primers and probes may be mixed to construct multiplex one-step fluorescence probe-based real-time PCR in one-tube with high specificity and high sensitivity.

According to a particular aspect, there are provided highly sensitive and specific primers, fragments and/or derivatives thereof useful in a method of PCR capable of detecting influenza A and/or B virus DNA in patient specimens. This test may be used to examine the specimens from patients with influenza A and/or B virus. The primers may be sensitive and specific. Further, at least one IC molecule may be included in each reaction to monitor the PCR performance.

### BRIEF DESCRIPTION OF THE FIGURES

Figure 1 is a graph showing the results of a 4-plex RT-PCR, detecting (A) (FAM) Flu A; (B) (HEX) Swine H1N1; (C) (TexRD) Flu B; (D) (CY5) Flu A H3N2
Figure 2 is a photograph of the results of an agarose gel electrophoresis showing the size of PCR products which were analysed by Ethidium bromide

### DETAILED DESCRIPTION OF THE PREFERRED EMBODIMENTS

Bibliographic references mentioned in the present specification are for convenience listed in the form of a list of references and added at the end of the examples.

### Definitions

The term "biological sample" is herein defined as a sample of any tissue and/or fluid from at least one animal and/or plant. Biological samples may be animal, including human, fluid, solid (e.g., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, lagamorphs, rodents, etc. Environmental samples include environmental material such as surface matter, soil, water, air and industrial samples, as well as samples obtained from food and dairy processing instruments, apparatus, equipment, utensils, disposable and non-disposable items. These examples are not to be construed as limiting the sample types applicable to the methods disclosed herein. In particular, a biological sample may be of any tissue and/or fluid from at least a human being.

The term "complementary" is used herein in reference to polynucleotides (i.e., a sequence of nucleotides such as an oligonucleotide or a target nucleic acid) related by the base-pairing rules. For example, for the sequence "5'-A-G-T-3'," is complementary to the sequence "3'-T-C-A-5'." The degree of complementarity between nucleic acid strands has significant effects on the efficiency and strength of hybridization between nucleic acid strands. This is of particular importance in amplification reactions, as well as detection methods that depend upon binding between nucleic acids. In particular, the "complementary sequence" refers to an oligonucleotide which, when aligned with the nucleic acid sequence such that the 5' end of one sequence is paired with the 3' end of the other, is in "anti-parallel association." Certain bases not commonly found in natural nucleic acids may be included in the nucleic acids disclosed herein and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs. Where a first oligonucleotide is complementary to a region of a target nucleic acid and a second oligonucleotide has complementary to the same region (or a portion of this region) a "region of overlap" exists along the target nucleic acid. The degree of overlap may vary depending upon the extent of the complementarity.

The term "comprising" is herein defined as "including principally, but not necessarily solely". Furthermore, the term "comprising" will be automatically read by the person skilled in the art as including "consisting of". The variations of the word "comprising", such as "comprise" and "comprises", have correspondingly varied meanings.

The term "derivative," is herein defined as the chemical modification of the oligonucleotides of the present invention, or of a polynucleotide sequence complementary to the oligonucleotides. Chemical modifications of a polynucleotide sequence can include, for example, replacement of hydrogen by an alkyl, acyl, or amino group.

The term "fragment" is herein defined as an incomplete or isolated portion of the full sequence of an oligonucleotide which comprises the active/binding site(s) that confers the sequence with the characteristics and function of the oligonucleotide. In particular, it may be shorter by at least one nucleotide or amino acid. More in particular, the fragment comprises the binding site(s) that enable the oligonucleotide to bind to influenza virus. For example, the fragment of the forward primer may comprise at least 10, 12, 15, 18 or 19 consecutive nucleotides of SEQ ID NO:1, SEQ ID NO:4, SEQ ID NO:7, SEQ ID NO:10, SEQ ID NO:13, or SEQ ID NO:16 and/or the reverse primer may comprise at least 10, 12, 15, 18, 19, 20, 22, or 24 consecutive nucleotides of SEQ ID NO:2, SEQ ID NO:5, SEQ ID NO:8, SEQ ID NO:11, SEQ ID NO:14, SEQ ID NO:17, or SEQ ID NO:18. More in particular, the fragment of the primer may be at least 15 nucleotides in length.

The term "internal control (IC) molecule" is herein defined as the *in vitro* transcribed oligonucleotide molecule which is co-amplified by the same primer set for influenza virus used in the method of the present invention. In particular, the IC may be mixed in the reaction mixture to monitor the performance of PCR to avoid false negative results. The probe to detect this IC molecule may be specific to the interior part of this molecule. This interior part may be artificially designed and may not occur in nature.

The term "influenza virus" as used in the context of the invention includes all subtypes of influenza viruses that fall under the categories of "avian influenza viruses" and "human influenza viruses". The influenza viruses may be influenza A, B or C viruses. In particular, the influenza A virus may include but are not limited to H1N1, H3N2, H5N1, H5N2, H5N8, H5N9, H7N2, H7N3, H7N4, H7N7, H9N2 and the like.

The term "mutation" is herein defined as a change in the nucleic acid sequence of a length of nucleotides. A person skilled in the art will appreciate that small mutations, particularly point mutations of substitution, deletion and/or insertion has little impact on the stretch of nucleotides, particularly when the nucleic acids are used as probes. Accordingly, the oligonucleotide(s) according to the present disclosure encompasses mutation(s) of substitution(s), deletion(s) and/or insertion(s) of at least one nucleotide. Further, the oligonucleotide(s) and derivative(s) thereof according to the present disclosure may also function as probe(s) and hence, any oligonucleotide(s) referred to herein also encompasses their mutations and derivatives. For example, if mutations occur at a few base positions at any primer hybridization site of the target gene, particularly to the 5'-terminal, the sequence of primers may not affect the sensitivity and the specificity of the primers.

The term "nucleic acid in the biological sample" refers to any sample that contains nucleic acids (RNA or DNA). In particular, sources of nucleic acids are biological samples including, but not limited to blood, saliva, cerebral spinal fluid, pleural fluid, milk, lymph, sputum and semen.

According to an aspect, the present disclosure provides at least one isolated oligonucleotide comprising, consisting essentially of, or consisting of at least one nucleotide sequence selected from the group consisting of: SEQ ID NO:1 to SEQ ID NO:19, fragment(s), derivative(s), mutation(s), and complementary sequence(s) thereof. The oligonucleotide may be capable of binding to and/or being amplified from influenza virus. The influenza virus may be selected from influenza A and/or B. In particular, the oligonucleotides may be useful as primers and/or probes and may be used in methods for specifically detecting influenza A and/or B in a sample containing either one or both strains of influenza and/or other unrelated viruses/microscopic organisms. These nucleotide sequences of the primers and probes of the present disclosure are designed to hybridize specifically to regions of the influenza A and influenza B genomes that are unique to the genome of each strain, but which are also conserved across many viruses within each strain.

The primers according to any aspect of the present disclosure may be used to distinguish the genotype of influenza A from influenza B. In particular, the primers according to the present disclosure may be used to distinguish the genotype of the different subtypes of influenza A virus. Even more in particular, the primers may be used to distinguish influenza A and/or influenza B virus with swine-origin H1N1 subtyping confirmation, seasonal H1N1, seasonal H3N2 and the like.

The influenza virus may be a drug-resistant strain. The drug resistant strain of influenza virus may be resistant to one or more drugs selected from the group consisting of: aantadine, oeltamivir, rimantadine, zanamivir and the like. The drug resistant strain of influenza virus may be a multi-drug resistant strain which may be resistant to a plurality of drugs.

The oligonucleotide sequence may be between 13 and 35 linked nucleotides in length and may comprise at least 70% sequence identity to any of the sequences selected from the group consisting of SEQ ID NO:1 to SEQ ID NO:19. A skilled person will appreciate that a given primer need not hybridize with 100% complementarity in order to effectively prime the synthesis of a complementary nucleic acid strand in an amplification reaction. A primer may hybridize over one or more segments such that intervening or adjacent segments are not involved in the hybridization event, (e.g., for example, a loop structure or a hairpin structure). In particular, the sequence of the oligonucleotide may have 80%, 85%, 90%, 95% or 98% sequence identity to any one of the sequences selected from the group consisting of SEQ ID NO:1 to SEQ ID NO:19.

An extent of variation of 70% to 100%, or any range therewithin, of the sequence identity is possible relative to the specific primer sequences disclosed. Determination of sequence identity is described in the following example: a primer 20 nucleotides in length which is identical to another 20 nucleotides in length primer having two non-identical residues has 18 of 20 identical residues (18/20 = 0.9 or 90% sequence identity). In another example, a primer 15 nucleotides in length having all residues identical to a 15 nucleotides segment of primer 20 nucleobases in length would have 15/20 = 0.75 or 75% sequence identity with the 20 nucleotides primer.

Percent homology, sequence identity or complementarity, can be determined by, for example, the Gap program (Wisconsin Sequence Analysis Package, Version 8 for UNIX, Genetics Computer Group, University Research Park, Madison WI), using default settings, which uses the algorithm of Smith and Waterman (Adv. Appl. Math., 1981, 2, 482-489). A skilled person is able to calculate percent sequence identity or percent sequence homology and able to determine, without undue experimentation, the effects of variation of primer sequence identity on the function of the primer in its role in priming synthesis of a complementary strand of nucleic acid for production of an amplification product.

According to another aspect, the present disclosure provides at least one pair of oligonucleotides comprising at least one forward primer and at least one reverse primer, wherein the forward primer comprises, consists essentially of or consists of at least one nucleotide sequence selected from the group consisting of SEQ ID NO:1, SEQ ID NO:4, SEQ ID NO:7, SEQ ID NO:10, SEQ ID NO:13, SEQ ID NO:16, fragment(s), derivative(s), mutation(s), and complementary sequence(s) thereof and the reverse primer comprises, consists essentially of or consists of at least one nucleotide sequence selected from the group consisting of SEQ ID NO:2, SEQ ID NO:5, SEQ ID NO:8, SEQ ID NO:11, SEQ ID NO:14, SEQ ID NO:17, SEQ ID NO:18, fragment(s), derivative(s), mutation(s), and complementary sequence(s) thereof.

According to another aspect, the present disclosure provides at least one set of oligonucleotides comprising a pair of oligonucleotides according to any aspect of the present disclosure and at least one probe.

The probe may comprise, consists essentially of or consists of the nucleotide sequence from the group consisting of SEQ ID NO:3, SEQ ID NO:6, SEQ ID NO:9, SEQ ID NO:12, SEQ ID NO:15 and SEQ ID NO:19, fragment(s), derivative(s), mutation(s), and complementary sequence(s) thereof may be capable of binding to the amplicon.

The probe may be labeled with a fluorescent dye at 5' and 3' ends thereof. Examples of the 5'-labeled fluorescent dye may include, but are not limited to, 6-carboxyfluorescein (FAM), hexachloro-6- carboxyfluorescein (HEX), tetrachloro-o-carboxyfluorescein, and Cyanine-5 (Cy5). Examples of the 3'-labeled fluorescent dye may include, but are not limited to, 5-carboxytetramethylrhodamine (TAMRA) and black hole quencher- 1,2,3 (BHQ-1,2,3).

The oligonucleotides of the present disclosure may be used in various nucleic acid amplification techniques known in the art, such as, for example, Polymerase Chain Reaction (PCR), Nucleic Acid Sequence Based Amplification (NASBA), Transcription-Mediated Amplification (TMA), Rolling Circle Amplification (RCA), Strand Displacement Amplification (SDA), thermophilic SDA (tSDA) or Ligation-Mediated Amplification (LMA). The oligonucleotides of the present disclosure may also be used in a variety of methods known to one of ordinary skill in the art for direct detection of influenza A and B without amplification through direct hybridization with viral nucleic acids, or to detect DNA or RNA copies of viral nucleic acids, or their complements.

The oligonucleotide according to any aspect of the present disclosure may be used in a method for the detection of influenza from either a clinical or a culture sample, wherein the clinical samples may include but are not limited to, nasopharyngeal, nasal and throat swabs as well as nasopharyngeal aspirates and washes. The clinical sample may undergo preliminary processing prior to testing to allow more efficient detection of the viral nucleic acid. For example, the sample may be collected and may be added to transport medium to stabilize the virus. Nasopharyngeal, nasal and throat swabs may be added to a transport medium. Nasopharyngeal aspirates and washes may or may not be stabilized by addition of transport medium. Once received at the testing laboratory, the virus may be inactivated and lysed to liberate the viral RNA. The nucleic acid may optionally then be extracted to remove potential inhibitors or other interfering agents of later assay steps. To perform the methods of the disclosure, viral nucleic acids may be mixed with components essential for specific detection of influenza A and/or influenza B.

According to a further aspect, the present disclosure provides at least one amplicon amplified from influenza using at least one forward primer and at least one reverse primer according to any aspect of the present disclosure .

According to one aspect, the present disclosure provides at least one method of detecting and/or quantitating the presence of influenza virus in a biological sample, the method comprising the steps of:
(a) providing at least one biological sample;
(b) contacting at least one oligonucleotide, pair of oligonucleotides or set of oligonucleotides according to any aspect of the present disclosure, with at least one nucleic acid in the biological sample, and/or with at least one nucleic acid extracted, purified and/or amplified from the biological sample; and
(c) detecting and/or quantitating any binding resulting from the contacting in step (b) whereby the influenza virus is present when binding is detected.

According to one aspect, the present disclosure provides at least one method of amplifying influenza A and/or B virus nucleic acid, wherein said method comprises carrying out a polymerase chain reaction using at least one forward primer according to any aspect of the present disclosure and a reverse primer according to any aspect of the present disclosure .

According to another aspect, the present disclosure provides at least one kit for the detection of influenza A and/or B virus, the kit comprising at least one oligonucleotide, pair of oligonucleotides or set of oligonucleotides according to any aspect of the present disclosure . The kit may be used by clinicians to detect human and avian influenza viruses in patients that are afflicted with influenza symptoms. Such kit will include one or more primer and probe sets for the detection of avian influenza A, influenza A, or human influenza B.

The primer and/or probe sets for the detection of avian influenza A, influenza A, or human influenza B may be found in Table 1.

**Table 1. Five primer and/or probe sets for the detection of avian influenza A, influenza A, and/or human influenza B. "Y" stands for nucleotide C/T, "K" stands for G/T and "R" stands for nucleotide A/G.**

| **Name** | **Sequence** | **SEQ ID NO:** |
|---|---|---|
| **SET 1** | | |
| Flu A, Forward Primer, 490:A145F | (5'-3')TTCTAACCGAGGTCGAAACGTA | 1 |
| Flu A, Reverse Primer, 488:A145R | (5'-3')ATTGGTCTTGTCTTTAGCCAYT | 2 |
| Flu A, Probe, 500:A145U (Fluorescent dye) | 5'-CCCCTCAAAGCCGAGATCGC 3' (Quencher) | 3 |

| **SET 2** | | |
|---|---|---|
| Swine-Origin H1N1 Set A, Forward Primer, 470:H1s138F | (5'-3')GGCTGCTTTGAATTTTACCAC | 4 |
| Swine-Origin H1N1 Set A, Reverse Primer, 471:H1s138R | (5'-3') TGTTGATTCCAGCTTTACCC | 5 |
| Swine-Origin H1N1 Set A, Probe, 475:138P (Fluorescent dye) | 5'-GCGATAACACGTGCATGGAAAGT-3' (Quencher) | 6 |

| **SET 3** | | |
|---|---|---|
| Swine-Origin H1N1 Set B, Forward Primer, 476:H1s142F | (5'-3') AACTTTGGACTACCACGA | 7 |
| Swine-Origin H1N1 Set B, Reverse Primer, 477:H1s142R | (5'-3')TTTGACACTTTCCATGCAC | 8 |
| Swine-Origin H1N1 Set B, Probe, 478:142U (Fluorescent dye) | 5'-ACGGCTGCTTTGAATTTTACCAC-3' (Quencher) | 9 |

| **SET 4** | | |
|---|---|---|
| Seasonal H1N1, Forward Primer, 468:H1h218F | (5'-3')CCTGTTATGCACATTTACAGC | 10 |
| Seasonal H1N1, Reverse Primer, 469:H1h218R | (5'-3')CTAAGATCCACCCGGCAAC | 11 |
| Seasonal H1N1, Probe, 472:H1h218P (Fluorescent dye) | 5'-CTAACAACTCGACCGACACTG-3' (Quencher) | 12 |

| **SET 5** | | |
|---|---|---|
| Seasonal H3N2, Forward Primer, 482:H3,116F | (5'-3')AATGACAACAGCACGGCAAC | 13 |
| Seasonal H3N2, Reverse Primer, 483:H3,116R | (5'-3')TGAACCAGCTCAGTAGCATT | 14 |
| Seasonal H3N2, Probe, 484:H3,116U (Fluorescent dye) | 5'-CCATGCAGTACCAAACGGAACGA-3' (Quencher) | 15 |

| **SET 6** | | |
|---|---|---|
| Flu B, Forward Primer, 494:B188F | (5'-3')TTTGGAGACACAATTGCCTA | 16 |
| Flu B, Reverse Primer 1, 495:B188R1 | (5'-3')AAGCAKATAGAGGCACCA | 17 |
| Flu B, Reverse Primer 2 496:B188R2 | (5'-3')AAGCAGATAGAGGCGCCA | 18 |
| Flu B, Probe, 497:B188L (Fluorescent dye) | 5'-TCAAATTCTTTCCCACCRAAC-3' (Quencher) | 19 |

In one aspect, the present disclosure provides at least one set of oligonucleotides comprising:
- a forward primer comprising, consisting essentially of or consisting of at least one nucleotide sequence of SEQ ID NO:1, fragment(s), derivative(s), mutation(s), or complementary sequence(s) thereof;
- a reverse primer comprising, consisting essentially of or consisting of at least one nucleotide sequence of SEQ ID NO:2, fragment(s), derivative(s), mutation(s), or complementary sequence(s) thereof; and
- a probe comprising, consisting essentially of or consisting of at least one nucleotide sequence of SEQ ID NO:3, fragment(s), derivative(s), mutation(s), or complementary sequence(s) thereof.

The set according to this aspect of the present disclosure may capable of binding to and/or detecting influenza A virus from a sample.

According to another aspect, the present disclosure provides at least one set of oligonucleotides comprising:
- a forward primer comprising, consisting essentially of or consisting of at least one nucleotide sequence of SEQ ID NO:4, fragment(s), derivative(s), mutation(s), or complementary sequence(s) thereof;
- a reverse primer comprising, consisting essentially of or consisting of at least one nucleotide sequence of SEQ ID NO:5, fragment(s), derivative(s), mutation(s), or complementary sequence(s) thereof; and
- a probe comprising, consisting essentially of or consisting of at least one nucleotide sequence of SEQ ID NO:6, fragment(s), derivative(s), mutation(s), or complementary sequence(s) thereof.

The set according to this aspect of the present disclosure may capable of binding to and/or detecting influenza A virus. The influenza A virus may be H1N1 subtype of Influenza A virus. The influenza A virus may be swine origin H1N1 subtype of Influenza A virus.

According to another aspect, the present disclosure provides at least one set of oligonucleotides comprising:
- a forward primer comprising, consisting essentially of or consisting of at least one nucleotide sequence of SEQ ID NO:7, fragment(s), derivative(s), mutation(s), or complementary sequence(s) thereof;
- a reverse primer comprising, consisting essentially of or consisting of at least one nucleotide sequence of SEQ ID NO:8, fragment(s), derivative(s), mutation(s), or complementary sequence(s) thereof; and
- a probe comprising, consisting essentially of or consisting of at least one nucleotide sequence of SEQ ID NO:9, fragment(s), derivative(s), mutation(s), or complementary sequence(s) thereof.

The set according to this aspect of the present disclosure may capable of binding to and/or detecting influenza A virus. The influenza A virus may be H1N1 subtype of Influenza A virus. The influenza A virus may be swine origin H1N1 subtype of Influenza A virus.

According to another aspect, the present disclosure provides at least one set of oligonucleotides comprising:
- a forward primer comprising, consisting essentially of or consisting of at least one nucleotide sequence of SEQ ID NO:10, fragment(s), derivative(s), mutation(s), or complementary sequence(s) thereof;
- a reverse primer comprising, consisting essentially of or consisting of at least one nucleotide sequence of SEQ ID NO:11, fragment(s), derivative(s), mutation(s), or complementary sequence(s) thereof; and
- a probe comprising, consisting essentially of or consisting of at least one nucleotide sequence of SEQ ID NO:12, fragment(s), derivative(s), mutation(s), or complementary sequence(s) thereof.

The set according to this aspect of the present disclosure may capable of binding to and/or detecting influenza A virus. The influenza A virus may be H1N1 subtype of Influenza A virus. The influenza A virus may be seasonal H1 N1.

According to one aspect, the present disclosure provides at least one set of oligonucleotides comprising:
- a forward primer comprising, consisting essentially of or consisting of at least one nucleotide sequence of SEQ ID NO:13, fragment(s), derivative(s), mutation(s), or complementary sequence(s) thereof;
- a reverse primer comprising, consisting essentially of or consisting of at least one nucleotide sequence of SEQ ID NO:14, fragment(s), derivative(s), mutation(s), or complementary sequence(s) thereof; and
- a probe comprising, consisting essentially of or consisting of at least one nucleotide sequence of SEQ ID NO:15, fragment(s), derivative(s), mutation(s), or complementary sequence(s) thereof.

The set according to this aspect of the present disclosure may capable of binding to and/or detecting influenza A virus. The influenza A virus may be H3N2 subtype of Influenza A virus. The influenza A virus may be swine origin H3N2 subtype of Influenza A virus.

According to another aspect, the present disclosure provides at least one set of oligonucleotides comprising:
- a forward primer comprising, consisting essentially of or consisting of at least one nucleotide sequence of SEQ ID NO:16, fragment(s), derivative(s), mutation(s), or complementary sequence(s) thereof;
- a reverse primer comprising, consisting essentially of or consisting of at least one nucleotide sequence of SEQ ID NO:17 or SEQ ID NO:18, fragment(s), derivative(s), mutation(s), or complementary sequence(s) thereof; and
- a probe comprising, consisting essentially of or consisting of at least one nucleotide sequence of SEQ ID NO:19, fragment(s), derivative(s), mutation(s), or complementary sequence(s) thereof.

The set according to this aspect of the present disclosure may capable of binding to and/or detecting influenza B virus.

According to one aspect, the present disclosure provides at least one method of amplifying influenza virus nucleic acid, wherein said method comprises carrying out a polymerase chain reaction using the oligonucleotides according to any aspect of the present disclosure.

The method according to any aspect of the present disclosure may further comprise a step of mixing an internal molecule (IC) and a probe specific to the IC with the biological sample. The use of the IC may improve the efficiency of the influenza diagnosis increasing the accuracy of results.

It is submitted that the same will be more readily understood through reference to the following examples which are provided by way of illustration, and are not intended to be limiting of the present invention.

### EXAMPLES

Standard molecular biology techniques known in the art and not specifically described were generally followed as described in Sambrook and Russel, Molecular Cloning: A Laboratory Manual, Cold Springs Harbor Laboratory, New York (2001).

### EXAMPLE 1

Having now generally described the invention, the same will be more readily understood through reference to the following examples which are provided by way of illustration, and are not intended to be limiting of the present invention.

### 4-plex detection

Fluorescent probe based real-time one-step RT-PCR was performed with the SuperScript™ III RT/Platinum® Taq Mix (catalog no. 11732-20, Invitrogen, USA) in a 25-µl reaction volume containing 2.5µl of RNA sample, four pairs of forward/reverse primers, each primer at a final concentration of 0.15 µM and all four probes at final concentration of 0.1µM in a thermal cycler. Thermal cycling was performed by Stratagene Mx3000P (Stratagene, La Jolla, USA). Fluorescence detection was then read at each cycle to reveal the positive samples. The four primer pairs included A145F/R (SEQ ID NO:1 and SEQ ID NO:2 respectively), H1s142F/R (SEQ ID NO:7 and SEQ ID NO:8 respectively), H3-116F/R (SEQ ID NO:13 and SEQ ID NO:14 respectively), and B188F/R1,R2 (SEQ ID NO:16, SEQ ID NO:17 and SEQ ID NO:18 respectively). The four corresponding fluorescent probes included A145-FAM (SEQ ID NO:3), H1s142-HEX (SEQ ID NO:9), H3-116-Cy5 (SEQ ID NO:15) and B188-TxRd (SEQ ID NO:19).

The Stratagene Mx3000P (Stratagene, La Jolla, USA) was used with the following steps and conditions: reverse transcription at 55°C for 10 min; initial activation of Platinum Taq DNA polymerase at 95°C for 2.5 min, followed by 42 cycles of: denaturation at 95°C for 17s, annealing at 55°C for 31s and extension at 68°C for 32s. Fluorescent data collection point was placed on 55°C plateau annealing step of each cycle.

RT-PCR was carried out on the following samples.
1. Swine-like H1N1, Purified RNA extracted from A/Auckland/3/2009 H1N1 swine like influenza by WHO, Australia.
   (-5): 10⁻⁵ dilution, (-6): 10⁻⁶ dilution, (-7); 10⁻⁷ dilution from the stock.
2. H3N2, Purified RNA extracted from a patient.
   (-2): 10⁻² dilution, (-3): 10⁻³ dilution.
3. Flu B, Purified RNA extracted from virus stock obtained from ATCC, VR-786.
   (-5):10⁻⁵ dilution, (-6): 10⁻⁶ dilution.
4. NTC, Water.

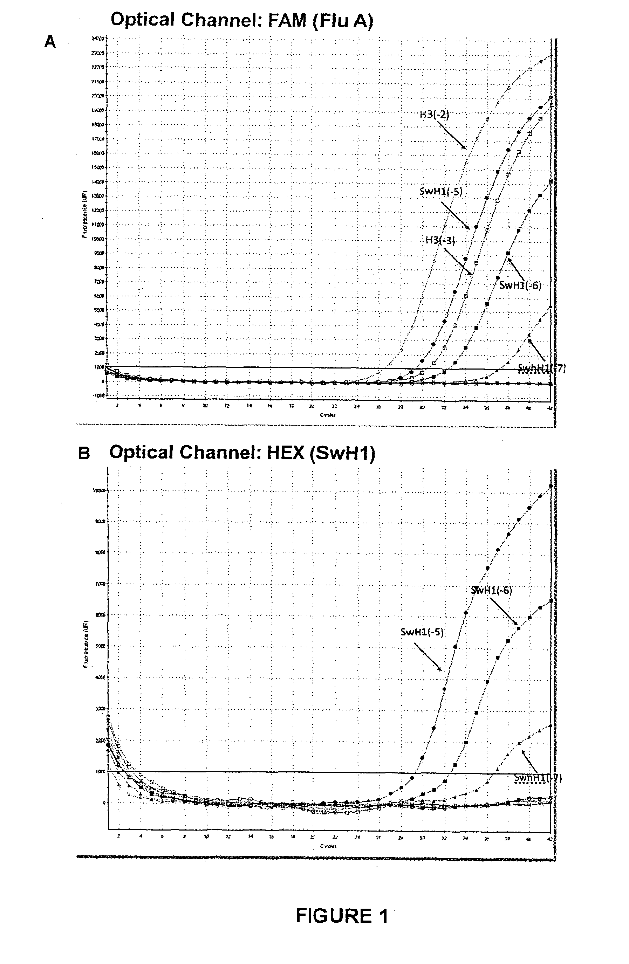

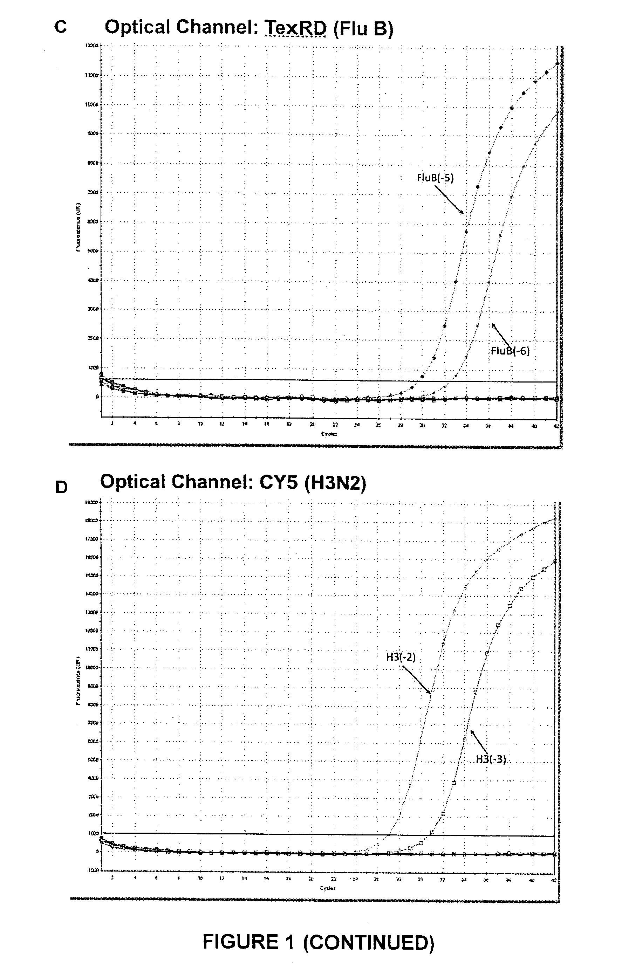

The results are shown in Table 2. The cycle threshold (Ct) was plotted against the number of cycles and shown Figure 1.

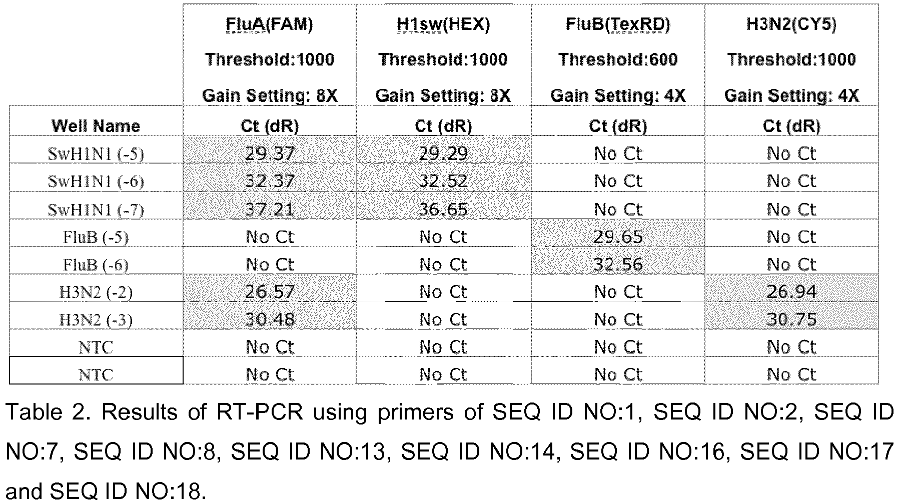

### EXAMPLE 2

### H1s138 primer pair (SEQ ID NO: 4 and SEQ ID NO:5) for gel electrophoresis

Monoplex one-step RT-PCR was performed with enzyme mixture, SuperScript® III Platinum® One-Step qRT-PCR Kit (Cat. No: 11732-20) containing a primer pair, H1s138 primer pair (i.e. SEQ ID NO:4 and SEQ ID NO:5). PCR products were analysed by Ethidium bromide - agarose gel electrophoresis.

A PCR was carried out with the following samples:
1. Swine-like H1N1, Purified RNA extracted from A/Auckland/3/2009 H1N1 swine like influenza by WHO, Australia. (-6): 10⁻⁶ dilution from the stock;
2. Patient samples; and
3. Negative control, Water.

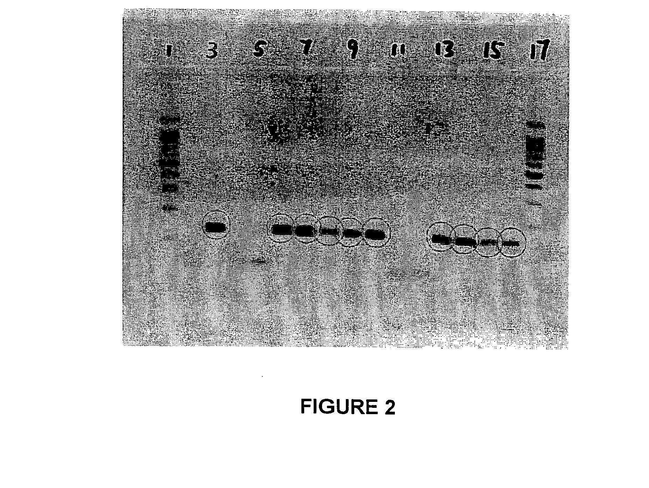

The results are shown in Table 3. The results of the electrophoresis is shown in Figure 2. The expected length of PCR product is 138 bp. As can be seen in Figure 2, the primers are specific to the target and resulted in a 138 bp band for the positive control and some patients' samples.

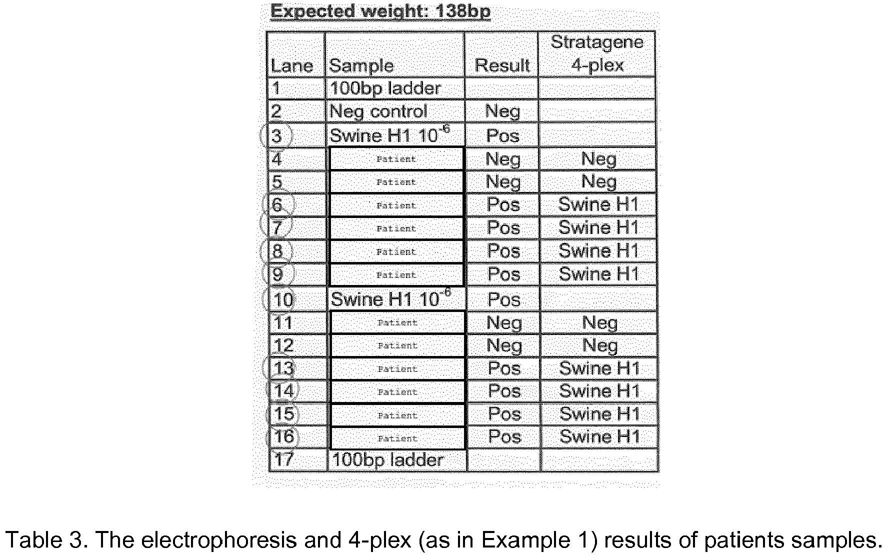

4-plex RT-PCR as explained in Example 1 was used on the same patients' samples. The results are shown in Table 2. As can be seen, the results of the electrophoresis corresponds with the results of the 4-plex RT-PCR as patients' samples that were positive in Figure 2 (i.e. showing a 138bp band), were confirmed to have swine origin H1N1.

### REFERENCES

Sambrook and Russel, Molecular Cloning: A Laboratory Manual, Cold Springs Harbor Laboratory, New York (2001).
Smith and Waterman Adv. Appl. Math., 1981, 2, 482-489.

## Claims

1. A kit for multiplex detection of influenza A or influenza A and influenza B virus in a biological sample, the kit comprising a mixture of primers and probes said mixture comprising a set of oligonucleotides comprising at least one forward primer and at least one reverse primer and at least one probe, wherein the forward primer comprises the nucleotide sequence of SEQ ID NO:1, the reverse primer comprises the nucleotide sequence of SEQ ID NO:2 and the probe comprises the nucleotide sequence of SEQ ID NO:3, and said mixture further comprising one or more sets of oligonucleotides selected from the group comprising:
a. a forward primer comprising the nucleotide sequence of SEQ ID NO: 4 and a reverse primer comprising the nucleotide sequence of SEQ ID NO: 5 and at least one probe comprising SEQ ID NO:6 to detect swine-origin H1N1;
b. a forward primer comprising the nucleotide sequence of SEQ ID NO: 7 and a reverse primer comprising the nucleotide sequence of SEQ ID NO: 8 and at least one probe comprising SEQ ID NO:9 to detect swine-origin H1N1;
c. a forward primer comprising the nucleotide sequence of SEQ ID NO: 10 and a reverse primer comprising the nucleotide sequence of SEQ ID NO: 11 and at least one probe comprising SEQ ID NO:12 to detect seasonal H1N1;
d. a forward primer comprising the nucleotide sequence of SEQ ID NO: 13 and a reverse primer comprising the nucleotide sequence of SEQ ID NO: 14 and at least one probe comprising SEQ ID NO:15 to detect seasonal H3N2, and
e. a forward primer comprising the nucleotide sequence of SEQ ID NO: 16 and a reverse primer comprising the nucleotide sequence selected from SEQ ID NO: 17 and 18 and at least one probe comprising or consisting of SEQ ID NO: 19 to detect influenza B.

2. The kit according to claim 1, wherein the kit comprises a mixture of primers and probes said mixture comprising the sets of oligonucleotides in a) or b), and d) to detect influenza A and influenza A virus subtypes swine-origin H1N1 and seasonal H3N2.

3. The kit according to claim 1, wherein the kit comprises a mixture of primers and probes said mixture comprising the sets of oligonucleotides in a) or b), c) and d) to detect influenza A and influenza A virus subtypes swine-origin H1N1, seasonal H1N1, and seasonal H3N2.

4. The kit according to claim 1, wherein the kit comprises a mixture of primers and probes said mixture comprising the sets of oligonucleotides in a) or b), d) and e) to detect influenza A and influenza A virus subtypes swine-origin H1N1, seasonal H3N2 and influenza B.

5. The kit according to claim 1, wherein the kit comprises a mixture of primers and probes said mixture comprising the sets of oligonucleotides in b), d) and e) to detect influenza A and influenza A virus subtypes swine-origin H1N1, seasonal H3N2 and influenza B.

6. The kit according to claim 1, wherein the kit comprises a mixture of primers and probes said mixture comprising the sets of oligonucleotides in a) or b), and c) to e) to detect influenza A and influenza A virus subtypes swine-origin H1N1, seasonal H1N1, seasonal H3N2 and influenza B.

7. The kit according to claim 1, wherein the kit comprises a mixture of primers and probes said mixture comprising the sets of oligonucleotides in b) to e) to detect influenza A and influenza A virus subtypes swine-origin H1N1, seasonal H1N1, seasonal H3N2 and influenza B.

8. An in vitro method of detecting and/or quantitating the presence of influenza A in a biological sample, the method comprising the steps of:
(a) providing at least one biological sample;
(b) contacting a mixture of primers and probes as defined in any one of claims 1 to 3, with at least one nucleic acid in the biological sample, and/or with at least one nucleic acid extracted, purified and/or amplified from the biological sample; and
(c) detecting and/or quantitating any binding resulting from the contacting in step (b) whereby the virus is present when binding is detected.

9. An in vitro method of detecting and/or quantitating the presence of Influenza A and B virus in a biological sample, the method comprising the steps of:
(a) providing at least one biological sample;
(b) contacting a mixture of primers and probes according to any one of claims 1 and 4 to 7, with at least one nucleic acid in the biological sample, and/or with at least one nucleic acid extracted, purified and/or amplified from the biological sample; and
(c) detecting and/or quantitating any binding resulting from the contacting in step (b) whereby the virus is present when binding is detected.

10. The use of a mixture of primers and probes for multiplex detection of influenza A or influenza A and influenza B virus in a biological sample said mixture comprising a set of oligonucleotides comprising at least one forward primer and at least one reverse primer and at least one probe, wherein the forward primer comprises the nucleotide sequence of SEQ ID NO:1, the reverse primer comprises the nucleotide sequence of SEQ ID NO:2 and the probe comprises the nucleotide sequence of SEQ ID NO:3, and said mixture further comprising one or more sets of oligonucleotides selected from the group comprising:
f. a forward primer comprising the nucleotide sequence of SEQ ID NO: 4 and a reverse primer comprising the nucleotide sequence of SEQ ID NO: 5 and at least one probe comprising SEQ ID NO:6 to detect swine-origin H1N1;
g. a forward primer comprising the nucleotide sequence of SEQ ID NO: 7 and a reverse primer comprising the nucleotide sequence of SEQ ID NO: 8 and at least one probe comprising SEQ ID NO:9 to detect swine-origin H1N1;
h. a forward primer comprising the nucleotide sequence of SEQ ID NO: 10 and a reverse primer comprising the nucleotide sequence of SEQ ID NO: 11 and at least one probe comprising SEQ ID NO:12 to detect seasonal H1N1;
i. a forward primer comprising the nucleotide sequence of SEQ ID NO: 13 and a reverse primer comprising the nucleotide sequence of SEQ ID NO: 14 and at least one probe comprising SEQ ID NO:15 to detect seasonal H3N2, and
j. a forward primer comprising the nucleotide sequence of SEQ ID NO: 16 and a reverse primer comprising the nucleotide sequence selected from SEQ ID NO: 17 and 18 and at least one probe comprising or consisting of SEQ ID NO: 19 to detect influenza B.

## Patentansprüche

1. Kit für Multiplex-Nachweis von Influenza A- oder Influenza A- und Influenza B-Virus in einer biologischen Probe, wobei der Kit eine Mischung aus Primern und Sonden umfasst, wobei die Mischung einen Oligonukleotidsatz umfasst, der wenigstens einen Vorwärts-Primer und wenigstens einen Umkehr-Primer und wenigstens eine Sonde umfasst, wobei der Vorwärts-Primer die Nukleotidsequenz von SEQ ID NO. 1 umfasst, der Umkehr-Primer die Nukleotidsequenz von SEQ ID NO: 2 umfasst und die Sonde die Nukleotidsequenz von SEQ ID NO: 3 umfasst, und die Mischung weiter einen oder mehrere Oligonukleotidsätze umfasst, der/die ausgewählt sind aus der Gruppe umfassend:
a. einen Vorwärts-Primer umfassend die Nukleotidsequenz von SEQ ID NO: 4 und einen Umkehr-Primer umfassend die Nukleotidsequenz von SEQ ID NO: 5 und wenigstens eine Sonde umfassend SEQ ID NO: 6, um H1N1 mit Ursprung vom Schwein nachzuweisen;
b. einen Vorwärts-Primer umfassend die Nukleotidsequenz von SEQ ID NO: 7 und einen Umkehr-Primer umfassend die Nukleotidsequenz von SEQ ID NO: 8 und wenigstens eine Sonde umfassend SEQ ID NO: 9, um H1N1 mit Ursprung vom Schwein nachzuweisen;
c. einen Vorwärts-Primer umfassend die Nukleotidsequenz von SEQ ID NO: 10 und einen Umkehr-Primer umfassend die Nukleotidsequenz von SEQ ID NO: 11 und wenigstens eine Sonde umfassend SEQ ID NO: 12, um jahreszeitlichen H1N1 nachzuweisen;
d. einen Vorwärts-Primer umfassend die Nukleotidsequenz von SEQ ID NO: 13 und einen Umkehr-Primer umfassend die Nukleotidsequenz von SEQ ID NO: 14 und wenigstens eine Sonde umfassend SEQ ID NO: 15, um jahreszeitlichen H3N2 nachzuweisen, und
e. einen Vorwärts-Primer umfassend die Nukleotidsequenz von SEQ ID NO: 16 und einen Umkehr-Primer umfassend die Nukleotidsequenz ausgewählt aus SEQ ID NO: 17 und 18 und wenigstens eine Sonde umfassend oder bestehend aus SEQ ID NO: 19, um Influenza B nachzuweisen.

2. Kit nach Anspruch 1, wobei der Kit eine Mischung aus Primern und Sonden umfasst, wobei die Mischung die Oligonukleotidsätze von a) oder b) und d) umfasst, um Influenza A und Influenza A-Virus-Subtypen H1N1 mit Ursprung vom Schwein und jahreszeitlichen H3N2 nachzuweisen.

3. Kit nach Anspruch 1, wobei der Kit eine Mischung aus Primern und Sonden umfasst, wobei die Mischung die Oligonukleotidsätze von a) oder b), c) und d) umfasst, um Influenza A und Influenza A-Virus-Subtypen H1N1 mit Ursprung vom Schwein, jahreszeitlichen H1N1 und jahreszeitlichen H3N2 nachzuweisen.

4. Kit nach Anspruch 1, wobei der Kit eine Mischung aus Primern und Sonden umfasst, wobei die Mischung die Oligonukleotidsätze von a) oder b), d) und e) umfasst, um Influenza A und Influenza A-Virus-Subtypen H1N1 mit Ursprung vom Schwein, jahreszeitlichen H3N2 und Influenza B nachzuweisen.

5. Kit nach Anspruch 1, wobei der Kit eine Mischung aus Primern und Sonden umfasst, wobei die Mischung die Oligonukleotidsätze von b), d) und e) umfasst, um Influenza A und Influenza A-Virus-Subtypen H1N1 mit Ursprung vom Schwein, jahreszeitlichen H3N2 und Influenza B nachzuweisen.

6. Kit nach Anspruch 1, wobei der Kit eine Mischung aus Primern und Sonden umfasst, wobei die Mischung die Oligonukleotidsätze von a) oder b) und c) bis e) umfasst, um Influenza A und Influenza A-Virus-Subtypen H1N1 mit Ursprung vom Schwein, jahreszeitlichen H1N1, jahreszeitlichen H3N2 und Influenza B nachzuweisen.

7. Kit nach Anspruch 1, wobei der Kit eine Mischung aus Primern und Sonden umfasst, wobei die Mischung die Oligonukleotidsätze von b) bis e) umfasst, um Influenza A und Influenza A-Virus-Subtypen H1N1 mit Ursprung vom Schwein, jahreszeitlichen H1N1, jahreszeitlichen H3N2 und Influenza B nachzuweisen.

8. In Vitro-Verfahren zum Nachweisen und/oder Quantifizieren der Anwesenheit von Influenza A in einer biologischen Probe, wobei das Verfahren die folgende Schritte umfasst:
(a) Bereitstellen wenigstens einer biologischen Probe;
(b) Kontaktieren einer Mischung aus Primern und Sonden, wie in einem der Ansprüche 1 bis 3 definiert, mit wenigstens einer Nukleinsäure in der biologischen Probe, und/oder mit wenigstens einer Nukleinsäure, die aus der biologischen Probe extrahiert, gereinigt und/oder amplifiziert ist; und
(c) Nachweisen und/oder Quantifizieren eines jeglichen Bindens, das sich aus dem Kontaktieren in Schritt (b) ergibt, wobei der Virus vorhanden ist, wenn Binden nachgewiesen wird.

9. In Vitro-Verfahren zum Nachweisen und/oder Quantifizieren der Anwesenheit von Influenza A- und B- Virus in einer biologischen Probe, wobei das Verfahren die folgende Schritte umfasst:
(a) Bereitstellen wenigstens einer biologischen Probe;
(b) Kontaktieren einer Mischung aus Primern und Sonden nach einem der Ansprüche 1 und 4 bis 7, mit wenigstens einer Nukleinsäure in der biologischen Probe, und/oder mit wenigstens einer Nukleinsäure, die aus der biologischen Probe extrahiert, gereinigt und/oder amplifiziert ist; und
(c) Nachweisen und/oder Quantifizieren eines jeglichen Bindens, das sich aus dem Kontaktieren in Schritt (b) ergibt, wobei der Virus vorhanden ist, wenn Binden nachgewiesen wird.

10. Verwendung einer Mischung aus Primern und Sonden für Multiplex-Nachweis von Influenza A- oder Influenza A- und Influenza B-Virus in einer biologischen Probe, wobei die Mischung einen Satz aus Oligonukleotiden umfasst, der wenigstens einen Vorwärts-Primer und wenigstens einen Umkehr-Primer und wenigstens eine Sonde umfasst, wobei der Vorwärts-Primer die Nukleotidsequenz von SEQ ID NO: 1 umfasst, der Umkehr-Primer die Nukleotidsequenz von SEQ ID NO: 2 umfasst und die Sonde die Nukleotidsequenz von SEQ ID NO: 3 umfasst, und die Mischung weiter einen oder mehrere Oligonukleotidsätze umfasst, die ausgewählt sind aus der Gruppe umfassend:
f. einen Vorwärts-Primer umfassend die Nukleotidsequenz von SEQ ID NO: 4 und einen Umkehr-Primer umfassend die Nukleotidsequenz von SEQ ID NO: 5 und wenigstens eine Sonde umfassend SEQ ID NO: 6, um H1N1 mit Ursprung vom Schwein nachzuweisen;
g. einen Vorwärts-Primer umfassend die Nukleotidsequenz von SEQ ID NO: 7 und einen Umkehr-Primer umfassend die Nukleotidsequenz von SEQ ID NO: 8 und wenigstens eine Sonde umfassend SEQ ID NO: 9, um H1N1 mit Ursprung vom Schwein nachzuweisen;
h. einen Vorwärts-Primer umfassend die Nukleotidsequenz von SEQ ID NO: 10 und einen Umkehr-Primer umfassend die Nukleotidsequenz von SEQ ID NO: 11 und wenigstens eine Sonde umfassend SEQ ID NO: 12, um jahreszeitlichen H1N1 nachzuweisen;
i. einen Vorwärts-Primer umfassend die Nukleotidsequenz von SEQ ID NO: 13 und einen Umkehr-Primer umfassend die Nukleotidsequenz von SEQ ID NO: 14 und wenigstens eine Sonde umfassend SEQ ID NO: 15, um jahreszeitlichen H3N2 nachzuweisen, und
j. einen Vorwärts-Primer umfassend die Nukleotidsequenz von SEQ ID NO: 16 und einen Umkehr-Primer umfassend die Nukleotidsequenz ausgewählt aus SEQ ID NO: 17 und 18 und wenigstens eine Sonde umfassend oder bestehend aus SEQ ID NO: 19, um Influenza B nachzuweisen.

## Revendications

1. Kit pour une détection multiplex de la grippe A ou du virus de la grippe A et de la grippe B dans un échantillon biologique, le kit comprenant un mélange d'amorces et de sondes, ledit mélange comprenant un ensemble d'oligonucléotides comprenant au moins une amorce sens et au moins une amorce inverse et au moins une sonde, où l'amorce sens comprend la séquence nucléotidique SEQ ID NO : 1, l'amorce inverse comprend la séquence nucléotidique SEQ ID NO : 2 et la sonde comprend la séquence nucléotidique SEQ ID NO : 3, et ledit mélange comprenant en outre un ou plusieurs ensemble(s) d'oligonucléotides choisis dans le groupe comprenant :
a. une amorce sens comprenant la séquence nucléotidique SEQ ID NO : 4 et une amorce inverse comprenant la séquence nucléotidique SEQ ID NO : 5 et au moins une sonde comprenant SEQ ID NO : 6 pour détecter la grippe H1N1 d'origine porcine ;
b. une amorce sens comprenant la séquence nucléotidique SEQ ID NO : 7 et une amorce inverse comprenant la séquence nucléotidique SEQ ID NO : 8 et au moins une sonde comprenant SEQ ID NO : 9 pour détecter la grippe H1N1 d'origine porcine ;
c. une amorce sens comprenant la séquence nucléotidique SEQ ID NO : 10 et une amorce inverse comprenant la séquence nucléotidique SEQ ID NO : 11 et au moins une sonde comprenant SEQ ID NO : 12 pour détecter la grippe H1N1 saisonnière ;
d. une amorce sens comprenant la séquence nucléotidique SEQ ID NO : 13 et une amorce inverse comprenant la séquence nucléotidique SEQ ID NO : 14 et au moins une sonde comprenant SEQ ID NO : 15 pour détecter la grippe H3N2 saisonnière, et
e. une amorce sens comprenant la séquence nucléotidique SEQ ID NO : 16 et une amorce inverse comprenant la séquence nucléotidique choisie parmi les séquences SEQ ID NO : 17 et 18 et au moins une sonde comprenant ou constituée SEQ ID NO : 19 pour détecter la grippe B.

2. Kit selon la revendication 1, dans lequel le kit comprend un mélange d'amorces et de sondes, ledit mélange comprenant les ensembles d'oligonucléotides dans a) ou b), et d) pour détecter la grippe A et les sous-types H1N1 d'origine porcine et H3N2 saisonnier du virus de la grippe A.

3. Kit selon la revendication 1, dans lequel le kit comprend un mélange d'amorces et de sondes, ledit mélange comprenant les ensembles d'oligonucléotides dans a) ou b), c) et d) pour détecter la grippe A et les sous-types H1N1 d'origine porcine, H1N1 saisonnier, et H3N2 saisonnier du virus de la grippe A.

4. Kit selon la revendication 1, dans lequel le kit comprend un mélange d'amorces et de sondes, ledit mélange comprenant les ensembles d'oligonucléotides dans a) ou b), d) et e) pour détecter la grippe A et les sous-types H1N1 d'origine porcine, H3N2 saisonnier du virus de la grippe A et la grippe B.

5. Kit selon la revendication 1, dans lequel le kit comprend un mélange d'amorces et de sondes, ledit mélange comprenant les ensembles d'oligonucléotides dans b), d) et e) pour détecter la grippe A et les sous-types H1N1 d'origine porcine, H3N2 saisonnier du virus de la grippe A et la grippe B.

6. Kit selon la revendication 1, dans lequel le kit comprend un mélange d'amorces et de sondes, ledit mélange comprenant les ensembles d'oligonucléotides dans a) ou b), et c) à e) pour détecter la grippe A et les sous-types H1N1 d'origine porcine, H1N1 saisonnier, H3N2 saisonnier du virus de la grippe A et la grippe B.

7. Kit selon la revendication 1, dans lequel le kit comprend un mélange d'amorces et de sondes, ledit mélange comprenant les ensembles d'oligonucléotides dans b) à e) pour détecter la grippe A et les sous-types H1N1 d'origine porcine, H1N1 saisonnier, H3N2 saisonnier du virus de la grippe A et la grippe B.

8. Procédé in vitro de détection et/ou de quantification de la présence de la grippe A dans un échantillon biologique, le procédé comprenant les étapes qui consistent :
(a) à fournir au moins un échantillon biologique ;
(b) à mettre en contact un mélange d'amorces et de sondes tel que défini dans l'une quelconque des revendications 1 à 3, avec au moins un acide nucléique dans l'échantillon biologique, et/ou avec au moins un acide nucléique extrait, purifié et/ou amplifié à partir de l'échantillon biologique ; et
(c) à détecter et/ou à quantifier toute liaison résultant de la mise en contact dans l'étape (b) moyennant quoi le virus est présent lorsque la liaison est détectée.

9. Procédé in vitro de détection et/ou de quantification de la présence de virus de la grippe A et de la grippe B dans un échantillon biologique, le procédé comprenant les étapes qui consistent :
(a) à fournir au moins un échantillon biologique ;
(b) à mettre en contact un mélange d'amorces et de sondes selon l'une quelconque des revendications 1 et 4 à 7, avec au moins un acide nucléique dans l'échantillon biologique, et/ou avec au moins un acide nucléique extrait, purifié et/ou amplifié à partir de l'échantillon biologique ; et
(c) à détecter et/ou à quantifier toute liaison résultant de la mise en contact dans l'étape (b) moyennant quoi le virus est présent lorsque la liaison est détectée.

10. Utilisation d'un mélange d'amorces et de sondes pour une détection multiplex de la grippe A ou de virus de la grippe A et de la grippe B dans un échantillon biologique, ledit mélange comprenant un ensemble d'oligonucléotides comprenant au moins une amorce sens et au moins une amorce inverse et au moins une sonde, où l'amorce sens comprend la séquence nucléotidique SEQ ID NO : 1, l'amorce inverse comprend la séquence nucléotidique SEQ ID NO : 2 et la sonde comprend la séquence nucléotidique SEQ ID NO : 3, et ledit mélange comprenant en outre un ou plusieurs ensemble(s) d'oligonucléotides choisis dans le groupe comprenant :
f. une amorce sens comprenant la séquence nucléotidique SEQ ID NO : 4 et une amorce inverse comprenant la séquence nucléotidique SEQ ID NO : 5 et au moins une sonde comprenant SEQ ID NO : 6 pour détecter la grippe H1N1 d'origine porcine ;
g. une amorce sens comprenant la séquence nucléotidique SEQ ID NO : 7 et une amorce inverse comprenant la séquence nucléotidique SEQ ID NO : 8 et au moins une sonde comprenant SEQ ID NO : 9 pour détecter la grippe H1N1 d'origine porcine ;
h. une amorce sens comprenant la séquence nucléotidique SEQ ID NO : 10 et une amorce inverse comprenant la séquence nucléotidique SEQ ID NO : 11 et au moins une sonde comprenant SEQ ID NO : 12 pour détecter la grippe H1N1 saisonnière ;
i. une amorce sens comprenant la séquence nucléotidique SEQ ID NO : 13 et une amorce inverse comprenant la séquence nucléotidique SEQ ID NO : 14 et au moins une sonde comprenant SEQ ID NO : 15 pour détecter la grippe H3N2 saisonnière, et
j. une amorce sens comprenant la séquence nucléotidique SEQ ID NO : 16 et une amorce inverse comprenant la séquence nucléotidique choisie parmi les séquences SEQ ID NO : 17 et 18 et au moins une sonde comprenant ou constituée SEQ ID NO : 19 pour détecter la grippe B.


### SEQUENCE LISTING

<110> Agency for science, Technology and Research
<120> INFLUENZA DETECTION METHOD AND KIT THEREFOR
<130> FP5107
<150> 200904777-0 <151> 2009-07-13
<160> 19
<170> PatentIn version 3.4
<210> 1
   <211> 22
   <212> DNA
   <213> Artificial
<220>
   <223> Forward Primer of Flu A (490:A145F)
<400> 1
   ttctaaccga ggtcgaaacg ta 22
<210> 2
   <211> 22
   <212> DNA
   <213> Artificial
<220>
   <223> Reverse Primer of Flu A (488:A145R)
<400> 2
   attggtcttg tctttagcca yt 22
<210> 3
   <211> 20
   <212> DNA
   <213> Artificial
<220>
   <223> Probe of Flu A (500:A145U)
<400> 3
   cccctcaaag ccgagatcgc 20
<210> 4
   <211> 21
   <212> DNA
   <213> Artificial
<220>
   <223> Forward Primer of Set A of Swine-Origin H1N1 (470:H1s138F)
<400> 4
   ggctgctttg aattttacca c 21
<210> 5
   <211> 20
   <212> DNA
   <213> Artificial
<220>
   <223> Reverse Primer of Set A of Swine-Origin H1N1(471:H1s138R)
<400> 5
   tgttgattcc agctttaccc 20
<210> 6
   <211> 23
   <212> DNA
   <213> Artificial
<220>
   <223> Probe of Set A of Swine-Origin H1N1 (475:138P)
<400> 6
   gcgataacac gtgcatggaa agt 23
<210> 7
   <211> 18
   <212> DNA
   <213> Artificial
<220>
   <223> Forward Primer of Set B of Swine-Origin H1N1 (476:H1s142F)
<400> 7
   aactttggac taccacga 18
<210> 8
   <211> 19
   <212> DNA
   <213> Artificial
<220>
   <223> Reverse Primer of Set B of Swine-Origin H1N1 (477:H1s142R)
<400> 8
   tttgacactt tccatgcac 19
<210> 9
   <211> 23
   <212> DNA
   <213> Artificial
<220>
   <223> Probe of Set B of Swine-Origin H1N1 (478:142U)
<400> 9
   acggctgctt tgaattttac cac 23
<210> 10
   <211> 21
   <212> DNA
   <213> Artificial
<220>
   <223> Forward Primer of Seasonal H1N1 (468:H1h218F)
<400> 10
   cctgttatgc acatttacag c 21
<210> 11
   <211> 19
   <212> DNA
   <213> Artificial
<220>
   <223> Reverse Primer of Seasonal H1N1 (469:H1h218R)
<400> 11
   ctaagatcca cccggcaac 19
<210> 12
   <211> 21
   <212> DNA
   <213> Artificial
<220>
   <223> Probe of Seasonal H1N1 (72:H1h218P)
<400> 12
   ctaacaactc gaccgacact g 21
<210> 13
   <211> 20
   <212> DNA
   <213> Artificial
<220>
   <223> Forward Primer of Seasonal H3N2 (482:H3,116F)
<400> 13
   aatgacaaca gcacggcaac 20
<210> 14
   <211> 20
   <212> DNA
   <213> Artificial
<220>
   <223> Reverse Primer of Seasonal H3N2 (483:H3,116R)
<400> 14
   tgaaccagct cagtagcatt 20
<210> 15
   <211> 23
   <212> DNA
   <213> Artificial
<220>
   <223> Probe of Seasonal H3N2 (484:H3,116U)
<400> 15
   ccatgcagta ccaaacggaa cga 23
<210> 16
   <211> 20
   <212> DNA
   <213> Artificial
<220>
   <223> Forward Primer of Flu B (494:B188F)
<400> 16
   tttggagaca caattgccta 20
<210> 17
   <211> 18
   <212> DNA
   <213> Artificial
<220>
   <223> Reverse Primer 1 of Flu B (495:B188R1)
<400> 17
   aagcakatag aggcacca 18
<210> 18
   <211> 18
   <212> DNA
   <213> Artificial
<220>
   <223> Reverse Primer 2 of Flu B (496:B188R2)
<400> 18
   aagcagatag aggcgcca 18
<210> 19
   <211> 21
   <212> DNA
   <213> Artificial
<220>
   <223> Probe of Flu B (497:B188L)
<400> 19
   tcaaattctt tcccaccraa c 21